# EUROPEAN PATENT SPECIFICATION

(11) **EP 1 553 896 B1**
(45) Date of publication and mention of the grant of the patent: **09.09.2009**
(21) Application number: 03779177.9
(22) Date of filing: 21.10.2003
(51) Int. Cl.: A61F 2/06

(54) **STENT WITH ECCENTRIC COATING**
STENT MIT EXZENTRISCHER BESCHICHTUNG
TUTEUR A REVETEMENT EXCENTRIQUE

(30) Priority: 22.10.2002 US 420773 P
(43) Date of publication of application: 20.07.2005
(73) Proprietor: Medtronic Vascular, Inc., Santa Rosa, CA 95403 (US)
(72) Inventor: FIFER, Dan, Windsor, CA 95492 (US); NOLTING, John, Poway, CA 92064 (US)
(74) Representative: Zimmermann, Gerd Heinrich
(86) International application number: PCT/US2003/033525
(87) International publication number: WO 2004/037126

(56) References cited:
- WO-A-99/32051
- US-A1- 2003 088 307

## Description

### TECHNICAL FIELD

The technical field of this disclosure is medical implant devices, particularly, coated stents.

### BACKGROUND OF THE INVENTION

Stents are generally cylindrical shaped devices that are radially expandable to hold open a segment of a blood vessel or other anatomical lumen after implantation into the body lumen. Stents have been developed with coatings to deliver drugs or other therapeutic agents.

Stents are used in conjunction with balloon catheters in a variety of medical therapeutic applications including intravascular angioplasty. For example, a balloon catheter device is inflated during PTCA (percutaneous transluminal coronary angioplasty) to dilate a stenotic blood vessel. The stenosis may be the result of a lesion comprised of, for example, a plaque or thrombus. After inflation, the pressurized balloon exerts a compressive force on the lesion thereby increasing the inner diameter of the affected vessel. The increased interior vessel diameter facilitates improved blood flow. Soon after the procedure, however, in a significant number of cases treated vessels re-narrow.

To reduce occurrence of restenosis, short flexible cylinders, or stents, constructed of metal or various polymers are implanted within the vessel to maintain lumen size. The stents act as a scaffold to support the lumen in an open position. Various configurations of stents include a cylindrical tube defined by a mesh, interconnected stents or like segments. Some exemplary stents are disclosed in U.S. Patent No. 5,292,331 to Boneau, U.S. Patent No. 6,090,127 to Globerman, U.S. Patent No. 5,133,732 to Wiktor, U.S. Patent No. 4,739,762 to Palmaz and U.S. Patent No. 5,421,955 to Lau. Balloon-expandable stents are mounted on a collapsed balloon at a diameter smaller than when the stents are deployed. Stents can also be self-expanding, growing to a final diameter when deployed without mechanical assistance from a balloon or like device.

Stents have been used with coatings to deliver drug or other therapy at the site of the stent. The coating may also be passive. The coating can be applied as a liquid containing the drug or other therapeutic agent dispersed in a polymer/solvent matrix. The liquid coating then dries to a solid coating upon the stent. The liquid coating can be applied by dipping or spraying the stent while spinning or shaking the stent to achieve a uniform coating. Combinations of the various application techniques can also be used.

The purpose of the coating is to deliver the drug to the tissue adjacent to the stent, such as the interior wall of an artery or vessel. Therefore, it is most important that the drug be present on at least the outer diameter of the stent. The coating has generally been applied uniformly over the inside and outside diameters of the stent, in one or more layers over the stent wires. Because drugs to reduce the occurrence of restenosis are only required at the stent outer diameter where the stent contacts the vessel tissue and not at the stent inner diameter containing blood, applying a drug-containing coating uniformly can lead to adverse drug effects or delivery to non-target tissue. For example, a drug on the inner diameter can flow into the blood where it is not beneficial. In addition, some drugs and polymers require a certain quantity or concentration before an effective therapy can be delivered. A uniform coating of a thickness necessary to provide sufficient drug at the outer diameter of the stent to constitute an effective therapy may interfere with blood flow through the inner lumen of the stent.

U.S. Patent No. 5,788,979 to Eckhard discloses a stent having an opening cylindrical structure having coatings applied to the outer an inner surfaces of the cylindrical structure that are intended for different purposes.

It is therefore an object of the present invention to provide a stent that would overcome the above disadvantages.

### SUMMARY OF THE INVENTION

This object is solved by a stent according to claim 1 and a method for producing a stent according to claim 14. Further aspects, features, details and advantages of the present invention are apparent from the dependent claims, the description and the accompanying drawings. One aspect of the present invention provides a stent having an eccentric coating to target drug delivery to tissue at the outer diameter of the stent.

Another aspect of the present invention provides a stent having an eccentric coating to avoid drug waste.

Another aspect of the present invention provides a stent having an eccentric coating to reduce drug delivery to the blood.

Another aspect of the present invention provides a stent having an eccentric coating able to provide different characteristics and therapies at the vessel wall and lumen sides.

The foregoing and other features and advantages of the invention will become further apparent from the following detailed description of the embodiments, read in conjunction with the accompanying drawings. The detailed description and drawings are merely illustrative of the invention, rather than limiting the scope of the invention being defined by the appended claims and equivalents thereof.

### BRIEF DESCRIPTION OF THE DRAWINGS

**FIG. 1** shows a stent delivery system made in accordance with the present invention.

**FIGS. 2** **&** **3** show a stent and a cross section, respectively, of a coated stent made in accordance with the present invention.

**FIGS. 4A, 4B, 4C** and **4D** show various cross-sections of stent wires with eccentric coatings made in accordance with the present invention.

**FIG. 5** shows a method of manufacturing a coated stent made in accordance with the present invention.

**FIGS. 6-8** show a method of manufacturing a stent made in accordance with the present invention using a fixture mandrel to regulate flow.

**FIG. 9** shows an alternate embodiment of a method of applying a coating to a stent.

**FIG. 10** shows yet another alternate embodiment of a method of applying a coating to a stent.

### DETAILED DESCRIPTION OF THE INVENTION

The stent with an eccentric coating of the present invention provides a coating having a different coating thickness on the stent outer diameter and stent inner diameter, i.e., an eccentric coating. The eccentric coating can be the primary carrier for a drug or other therapeutic agent. The eccentric coating can be thicker on the stent outer diameter to supply more drug to the vessel wall in which the coated stent is deployed and less drug to the vessel lumen. In one embodiment, a cap coating can be disposed on the eccentric coating to protect the eccentric coating, control the elution rate from the eccentric coating, provide an additional drug carrier, or provide combinations thereof. The eccentric coating can be applied by spraying a coating liquid on the stent outer diameter with a fixture mandrel interior to the stent to regulate the spray to the stent inner diameter.

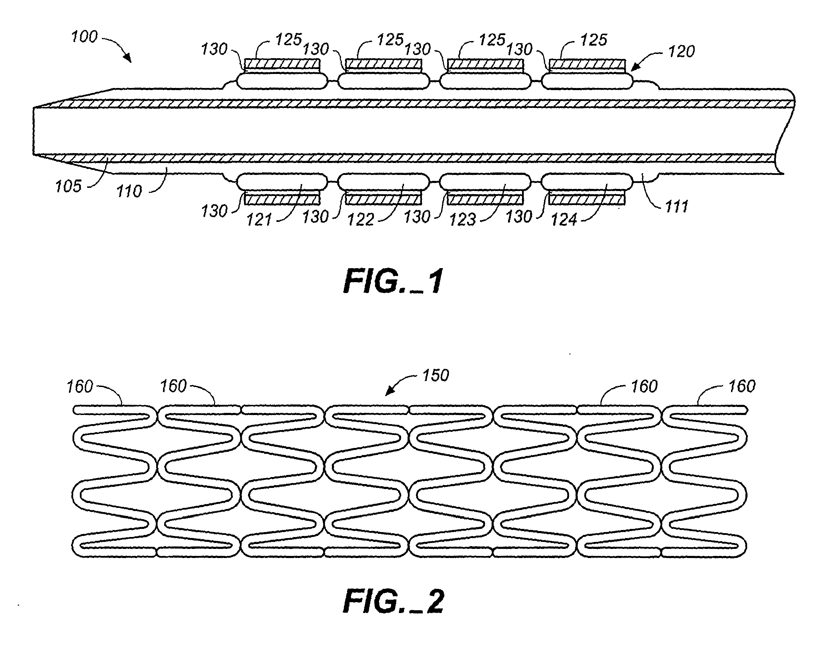

**FIG. 1** shows a stent delivery system made in accordance with the present invention. The stent delivery system **100** includes a catheter **105,** a balloon **110** operably attached to the catheter **105,** and a stent **120** disposed on the balloon **110.** The balloon **110,** shown in a collapsed state, may be any variety of balloons capable of expanding the stent **120.** The balloon **110** may be manufactured from any sufficiently elastic material such as polyethylene, polyethylene terephthalate (PET), nylon, or the like. In one embodiment, the balloon **110** may include retention means **111,** such as mechanical or adhesive structures, for retaining the stent **120** until it is deployed. The catheter **105** may be any variety of balloon catheters, such as a PTCA (percutaneous transluminal coronary angioplasty) balloon catheter, capable of supporting a balloon during angioplasty.

The stent **120** may be any variety of implantable prosthetic devices capable of carrying a coating known in the art. In one embodiment, the stent **120** may have a plurality of identical cylindrical stent segments placed end to end. Four stent segments **121, 122, 123,** and **124** are shown, and it will be recognized by those skilled in the art that an alternate number of stent segments may be used. The stent **120** includes at least one cap coating **125,** which can be applied to the stent **120** by dipping or spraying the stent **120** with a coating liquid, or applying the coating liquid with a combination of methods. The cap coating **125** can be applied as a liquid polymer/solvent matrix. A therapeutic agent can be incorporated in the cap coating **125,** or can be omitted and the cap coating **125** included for its mechanical properties alone.

An eccentric coating **130** between the cap coating **125** and the stent 120 is the primary carrier for a therapeutic agent. The cap coating **125** can be applied as a liquid containing the drug or other therapeutic agent dispersed in a polymer/solvent matrix.

The cap coating **125** and eccentric coating **130** are merely exemplary, and it should be recognized that other coating configurations, such as multiple coating layers, are possible. Although the cap coating **125** and the eccentric coating **130** are shown schematically on the outer circumference of the stent **120,** the cap coating **125** and the eccentric coating **130** can coat the whole stent **120,** both inside and outside, and around the cross section of individual stent wires. In another embodiment, the eccentric coating **130** can be present on a portion of the stent **120** without a cap coating **125** on that same portion.

The cap coating **125** and eccentric coating **130** can be a polymer including, but not limited to, urethane, polyester, epoxy, polycaprolactone (PCL), polymethylmethacrylate (PMMA), PEVA, PBMA, PHEMA, PEVAc, PVAc, Poly N-Vinyl pyrrolidone, Poly (ethylene-vinyl alcohol), combinations of the above, and the like. Suitable solvents that can be used to form the liquid coating include, but are not limited to, acetone, ethyl acetate, tetrahydrofuran (THF), chloroform, N-methylpyrrolidone (NMP), combinations of the above, and the like. Suitable therapeutic agent include, but are not limited to, antiangiogenesis agents, antiendothelin agents, antimitogenic factors, antioxidants, antiplatelet agents, antiproliferative agents, antisense oligonucleotides, antithrombogenic agents, calcium channel blockers, clot dissolving enzymes, growth factors, growth factor inhibitors, nitrates, nitric oxide releasing agents, vasodilators, virus-mediated gene transfer agents, agents having a desirable therapeutic application, combinations of the above, and the like. Specific example of therapeutic agents include abciximab, angiopeptin, colchicine, eptifibatide, heparin, hirudin, lovastatin, methotrexate, rapamycin, streptokinase, taxol, ticlopidine, tissue plasminogen activator, trapidil, urokinase, and growth factors VEGF, TGF-beta, IGF, PDGF, and FGF.

**FIG. 2** shows a coated stent made in accordance with the present invention. The stent **150** comprises a number of segments **160.** The pattern of the segments **160** can be W-shaped or can be a more complex shape with the elements of one segment continuing into the adjacent segment. The stent 150 can be installed in the stent delivery system of **FIG. 1** for implantation in a body lumen.

Referring to **FIG. 2****,** the stent **150** is conventional to stents generally and can be made of a wide variety of medical implantable materials, such as stainless steel (particularly 316-L stainless steel or 316LS), nitinol, tantalum, ceramic, nickel, titanium, aluminum, polymeric materials, tantalum, MP35N, titanium ASTM F63-83 Grade 1, niobium, high carat gold K 19-22, and combinations thereof. The stent **150** can be formed through various methods as well. The stent **150** can be welded, laser cut, molded, or consist of filaments or fibers which are wound or braided together in order to form a continuous structure. Depending on the material, the stent can be self expanding, or be expanded by a balloon or some other device. The cap coating and eccentric coating can be on the surface of the segments **160.**

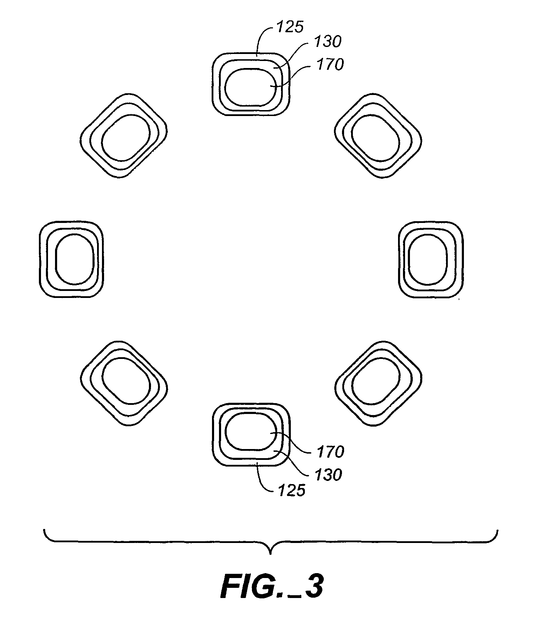

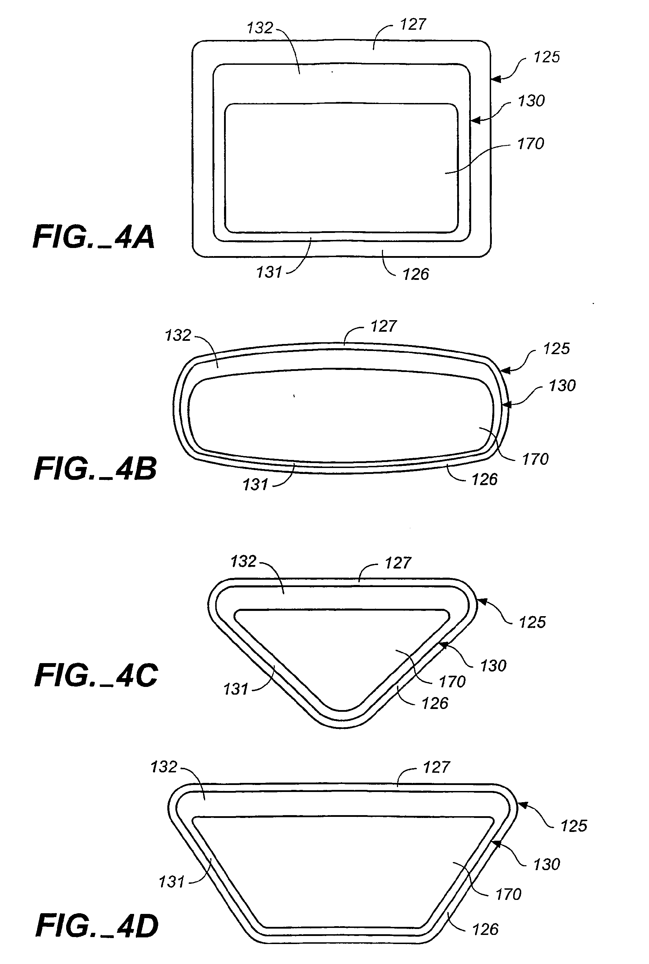

**FIG. 3** shows a cross section of a coated stent made in accordance with the present invention. A plurality of stent wires **170** are provided with a cap coating **125** and eccentric coating **130.** The stent wires form the segments, which form the stent. Although the cross section of the stent wires **170** is shown as generally rectangular with rounded corners, the cross section can be any number of shapes, for example and without limitation, those shown in **FIGS. 4A, 4B, 4C** and **4D****,** depending on fabrication methods, materials, and desired effect.

**FIGS. 4A, 4B, 4C** and **4D****,** in which like elements share like reference numbers with **FIG. 3****,** show examples of cross sections of stent wires with eccentric coatings made in accordance with the present invention. The cross sections can be of any number of other shapes depending on fabrication methods, materials, and desired effect. Please note, this is a representation only of the relative eccentricity of the stent coating - the actual stent cross section will vary depending on the type of stent used. An eccentric coating **130** is disposed on the stent wire **170** and a cap coating **125** can be disposed on the eccentric coating **130.** The eccentric coating **130** can comprise a first eccentric portion **131** positioned on the surface of the stent directly defining the inner lumen of the stent, i.e disposed towards the inner diameter of the stent and a second eccentric portion **132** positioned on the outer surface of the stent, i.e. disposed towards the outer diameter of the stent. The cap coating **125** can comprise a first cap portion **126** towards the stent inner diameter and a second cap portion **127** towards the stent outer diameter. In an alternate embodiment, the cap coating can be omitted and the eccentric coating provided as the outer layer of the stent.

The eccentric coating **130** can be the primary carrier of the therapeutic agent or drug. To concentrate the therapeutic agent at the vessel wall, rather than the vessel lumen, the second eccentric portion **132** can be thicker and have a greater volume than the first eccentric portion **131.** In an alternate embodiment, the second eccentric portion **132** can be thinner and have a lesser volume than the first eccentric portion **131** to concentrate the therapeutic agent at the vessel lumen for dispersion in the blood.

The cap coating **125** can be used for a number of purposes, including, but not limited to, a diffusion barrier to control the elution rate of the therapeutic agent from the eccentric coating **130;** a protective barrier to prevent damage to the eccentric coating **130;** a drug carrier for the same drug as the eccentric coating **130** or a different drug; a lubricating layer to reduce friction, or a binding layer to increase friction, between the stent and the balloon of the stent delivery system; or various combinations thereof. In one embodiment, the cap coating **125** can be of a single material and uniform thickness to form a concentric cap coating. Such a uniform coating can be produced by dipping the stent in a liquid coating. In another embodiment, the first cap portion **126** and second cap portion **127** can be of different thicknesses or of different materials to provide a number of useful combinations. For example, the second cap portion **127** can be thicker than the first cap portion **126,** paralleling the relative thickness of the second eccentric portion **132** and the first eccentric portion **131,** respectively. In another example, the second cap portion **127** can be rapidly biodegradable, while the first cap portion **126** is non-biodegradable, so that the vessel wall is exposed to the second eccentric portion **132** and the drug eluting there from, but the first cap portion **126** limits drug elution from the first eccentric portion **131** into the vessel lumen. In another example, the second cap portion **127** can be made of one polymer-drug combination of benefit to and compatible with the vessel wall and the first cap portion **126** made of a different polymer-drug combination beneficial to and compatible with the blood stream. In another example, the second eccentric portion **132** is of greater thickness than the first eccentric portion **131,** and the first cap portion **126** is of greater thickness than the second cap portion **127**, allowing for greater release of drug to the vessel wall while inhibiting drug release into the vessel lumen. Those skilled in the art will appreciate that many useful combinations of the first cap portion **126** and the second cap portion **127,** not limited to the examples presented herein, are possible. Additionally, in cases where the cap coating **125** incorporates a drug, a protective, or "sacrificial," biodegradable coating may be applied over the cap coating **125** to protect the cap coating **125** and the underlying eccentric coating **130** from damage during handling or deployment of the stent.

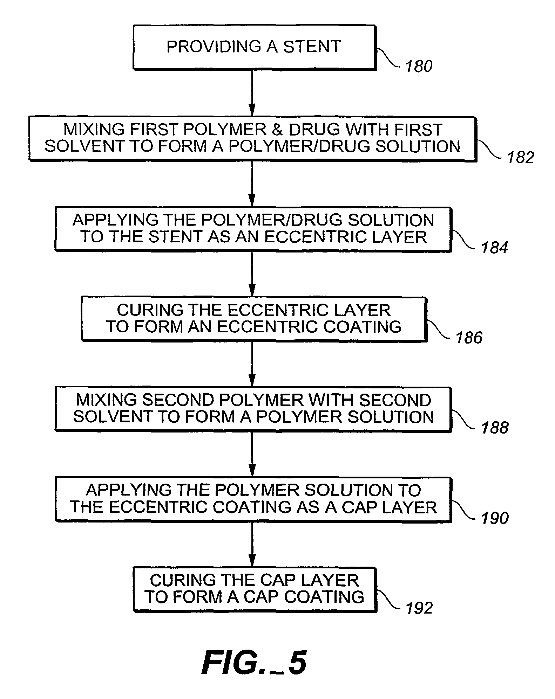

**FIG. 5** shows a method of manufacturing a coated stent made in accordance with the present invention. At **180,** a stent is provided. A first polymer and drug (or other therapeutic agent) are mixed with a first solvent to form a polymer/drug solution **182.** The polymer/drug solution is applied to the stent in an eccentric layer **184** and the eccentric layer cured to form an eccentric coating **186.** A second polymer is mixed with a second solvent to form a polymer solution **188.** The polymer solution is applied to the eccentric coating in a cap layer **190** and the cap layer cured to form a cap coating **192.** With reference to **FIG. 5****,** the use of the terms "polymer," "solvent" and "drug" in the singular shall, in each case, include them in the plural (i.e., mixtures of one or more polymers, solvents and/or drugs).

Those skilled in the art will appreciate that the method of manufacturing can be varied for the materials used and the results desired. For certain polymer/drug solutions and polymer solutions, the curing step can be omitted or can be a simple drying process. In another embodiment, the first polymer and first solvent can be the same combination as the second polymer and second solvent. In yet another embodiment, the polymer solution can also contain a drug or other therapeutic agent.

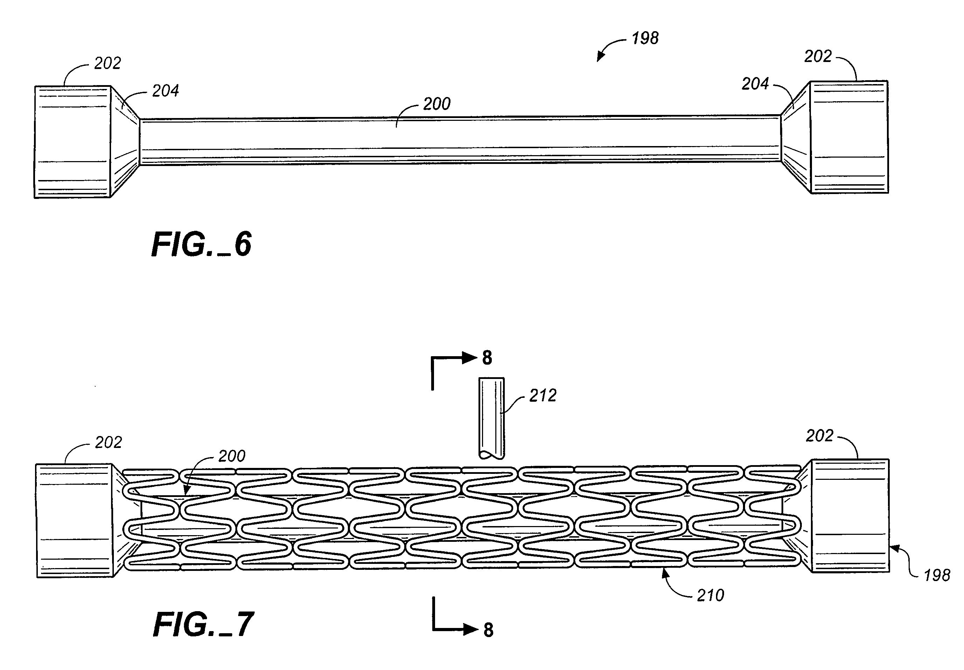

**FIGS. 6-8** show a method of manufacturing a coated stent made in accordance with the present invention using a fixture mandrel to regulate flow. Referring to **FIG. 6****,** a coating fixture **198** holds a stent while the coating is applied. The coating fixture **198** comprises a fixture mandrel **200** held in place by retainers **202.** The sloping face **204** helps to center and secure the stent during the coating operation. At least one of the retainers **202** can be attached to a drive (not shown) to rotate the stent in a single direction, or back and forth, during the coating operation. The drive can also provide axial movement if desired.

The fixture mandrel **200** can be held between the sloping faces **204** of the retainers **202,** or can be fixed to the retainers **202** by threads or other means. At least one of the retainers **202** can be detachable from the retainers **202** to allow installation of the stent to be coated between the retainers **202.** The fixture mandrel **200** can be metal or other material to block or redirect spray directed at the stent. The fixture mandrel **200** can be perforated to allow a portion of the spray to pass through the fixture mandrel **200.** The fixture mandrel **200** can be made of a sponge-like material or have a sponge-like outer coating to retain spray directed at the stent.

**FIG. 7****,** in which like elements share like reference numbers with **FIG. 6****,** shows a stent mounted on the coating fixture. The coating fixture **198** holds a stent **210** for application of the coating from spray nozzle **212.** To apply the coating over the whole stent **210,** the spray nozzle **212** can be attached to a drive (not shown) to move the spray nozzle **212** relative to the coating fixture **198.** The spray rate from the spray nozzle **212,** the movement of the spray nozzle **212,** and movement of the coating fixture **198** can be controlled by computerized numerically controlled machines to control the coating pattern and thickness on the stent **210.** The stent **210** can be a bare frame of metal or other stent base materials, or can be a stent with one or more coatings previously applied. Alternatively, the stent **210** and coating fixture **198** can move relative to a stationary spray nozzle **212,** or both the stent **210/** coating fixture **198** and the spray nozzle **212** can move relative to one another.

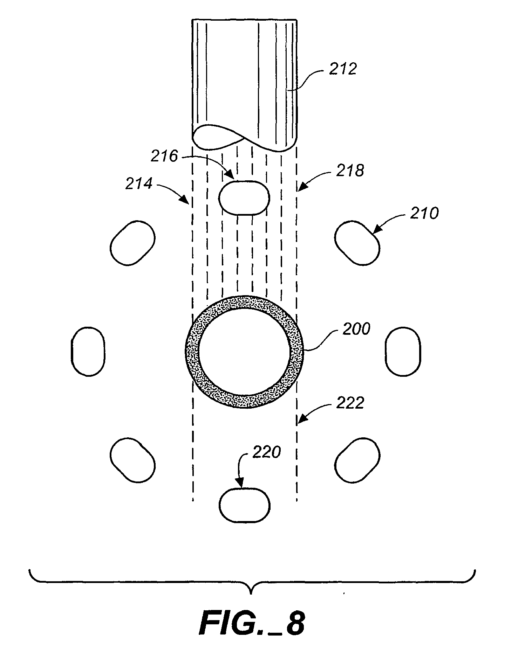

**FIG. 8****,** in which like elements share like reference numbers with **FIG. 6 & 7****,** shows a cross section of a stent mounted on the coating fixture during coating application. The spray nozzle **212** directs a spray **214** toward the stent **210.** The stent outer diameter **216** receives the full spray **218.** The fixture mandrel **200,** as well as the struts or wires of the stent **210** themselves, blocks a portion of the spray, so that the stent inner diameter **220** receives a shadow spray **222.** The spray rate difference between the full spray **218** and the shadow spray **222** results in a thicker coating on the stent outer diameter **216** than the stent inner diameter **220,** i.e., an eccentric coating. The stent **210** can be rotated in a single direction or in alternating directions to achieve coating characteristics as desired, depending on the coating materials used. The fixture mandrel **200** can optionally rotate with the stent.

The fixture mandrel **200** is shown as circular for purposes of illustration, but can be other shapes as desired. The fixture mandrel **200** can be perforated to allow spray to pass through the fixture mandrel **200** and reduce the spray rate difference between the full spray **218** and the shadow spray **222.** In another embodiment, the fixture mandrel **200** can be made of or coated with a sponge-like material to partially retain the spray and to reduce splatter from the surface of the fixture mandrel **200.** In yet another embodiment, the cross section of the fixture mandrel **200** can vary axially to vary the coating eccentricity of the coating with axial position. For example, the middle of the fixture mandrel can have a larger cross section to make the coating more eccentric (more coating on the stent outer diameter) at the middle of the stent, allowing a greater drug loading in the middle. Similarly, the ends of the fixture mandrel can be wider to make the coating more eccentric at the ends of the stent. In other embodiments a specific, uniform degree of eccentricity of the coating along the longitudinal axis of the stent can be determined by setting a specific, uniform cross section of the fixture mandrel.

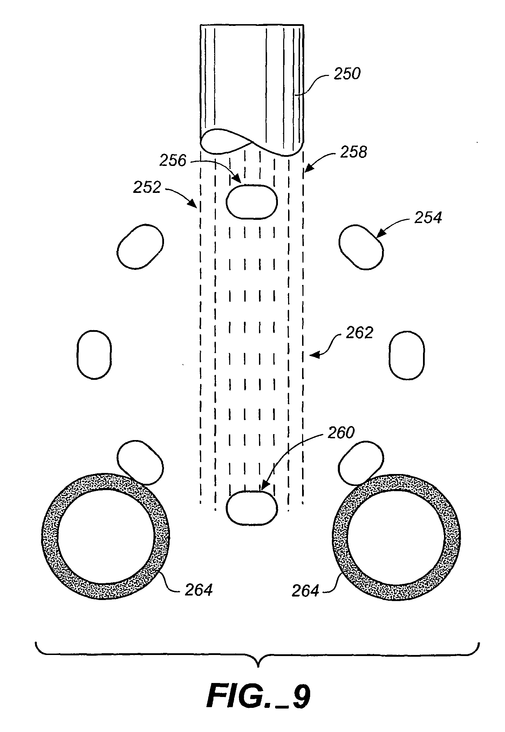

**FIG. 9** shows an alternate embodiment of a method of applying a coating to a stent. The spray nozzle **250** directs a spray **252** toward the stent **254.** The stent **254** can be a bare frame of metal or other stent base materials, or can be a stent with one or more coatings previously applied. The stent outer diameter **256** receives the full spray **258.** The stent outer diameter **256** blocks a portion of the spray, so that the stent inner diameter **260** receives a shadow spray **262.** The spray rate difference between the full spray **258** and the shadow spray **262** depends on the geometry between the spray nozzle **250** and the stent outer diameter **256,** including the proximity of the spray nozzle to the outside diameter and the size of the spray nozzle aperture.

In one embodiment, where the stent outer diameter **256** blocks a substantial portion of the spray, the spray rate difference between the full spray **258** and the shadow spray **262** results in a thicker coating on the stent outer diameter **216** than the stent inner diameter **220,** i.e., an eccentric coating. In another embodiment, where the stent outer diameter **256** blocks little of the spray, the spray rate difference between the full spray **258** and the shadow spray **262** results in substantially equal coating thicknesses on the stent outer diameter **216** and the stent inner diameter **220,** i.e., a uniform coating. The stent **210** can be rotated by fixture rollers **264** in a single direction or in alternating directions to achieve coating characteristics as desired, depending on the coating materials used. The fixture rollers **264** can be used alone or can be used in conjunction with the retainers described in **FIG. 7****.**

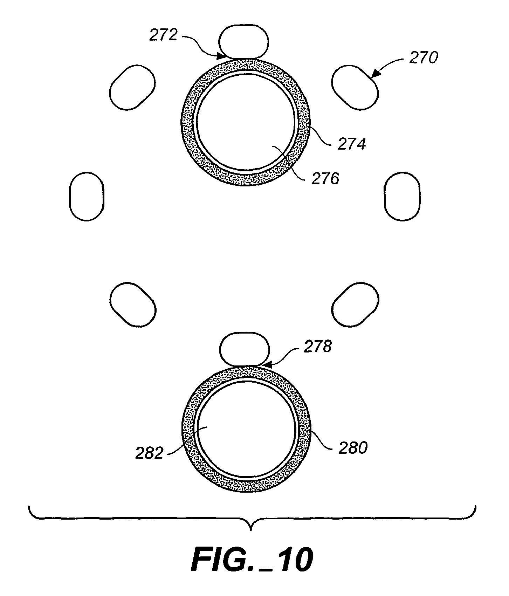

**FIG. 10** shows yet another alternate embodiment of a method of applying a coating to a stent. A pad carrying the liquid coating and disposed about a transfer roller transfers the liquid coating to the stent **270.** The stent **270** can be a bare frame of metal or other stent base materials, or can be a stent with one or more coatings previously applied. To coat the stent inner diameter **272,** inner pad **274** disposed about inner transfer roller **276** rotates against the inner diameter of the stent **270.** Typically, the inner transfer roller **276** and stent **270** can rotate in the same direction, but for certain stent and pad finishes, and liquid coating consistencies, the transfer roller **276** and stent **270** can rotate in opposite directions. To coat the stent outer diameter **278,** the outer pad **280** disposed about outer transfer roller **282** rotates against the outer diameter of the stent **270.** Typically, the outer transfer roller **282** and stent **270** can rotate in the opposite directions, but for certain stent and pad finishes, and liquid coating consistencies, the transfer roller **276** and stent **270** can rotate in opposite directions.

Although **FIG. 10** provides the example of applying the liquid coating to the stent inner and outer diameters simultaneously, the stent inner diameter and stent outer diameter could be coated in different steps. For example, the stent outer diameter can be coated to one thickness with one liquid coating, cured as necessary, and then the stent inner diameter coated with a different liquid coating to a different thickness. In another embodiment, the stent outer diameter could be coated with a biodegradable coating and the stent inner diameter could be coated with a non-biodegradable coating. This could allow the cap coating near the vessel wall to degrade quickly to permit underlying therapeutic agents to work at the vessel wall, while reducing the therapeutic agents entering the blood. Those skilled in the art will appreciate that many combinations of stent inner and outer diameter coating thicknesses, therapeutic agents, and coating materials can be used to advantage as desired.

It is important to note that **FIGS. 1-10** illustrate specific applications and embodiments of the present invention, and is not intended to limit the scope of the present disclosure or claims to that which is presented therein. For example, the cap coating and eccentric coating can be applied in a variety of conventional ways, including painting, spraying, dipping, wiping, electrostatic deposition, vapor deposition, epitaxial growth, combinations thereof, and other methods known to those of ordinary skill in the art. The means of applying the liquid coating, such as spray nozzles or pads, can be moved in various paths relative to the stent to achieve particular patterns and thickness variations. Upon reading the specification and reviewing the drawings hereof, it will become immediately obvious to those skilled in the art that myriad other embodiments of the present invention are possible, and that such embodiments are contemplated and fall within the scope of the presently claimed invention.

While the embodiments of the invention disclosed herein are presently considered to be typical, various changes and modifications can be made without departing from the scope of the invention. The scope of the invention is indicated in the appended claims, and all changes that come within the meaning and range of equivalents are intended to be embraced therein.

## Claims

1. A coated stent comprising:
a stent (120), the stent having a stent inner diameter and a stent outer diameter; and
an eccentric coating (130) comprising a continuous eccentric layer obtained by applying a polymer/drug solution to the stent, the eccentric coating (130) having a first eccentric portion (131) and a second eccentric portion (132), the first eccentric portion (131) disposed on the stent inner diameter and the second eccentric portion (132) disposed on the stent outer diameter;
wherein the first eccentric portion (131) and the second eccentric portion (132) have different thicknesses.

2. The coated stent of claim 1 wherein the second eccentric portion (132) is thicker than the first eccentric portion (131).

3. The coated stent of claim 1 wherein the first eccentric portion (131) is thicker than the second eccentric portion (132).

4. The coated stent of any of the preceding claims wherein the eccentric coating (130) includes a therapeutic agent.

5. The coated stent of claim 4 further comprising a cap coating (125) disposed on the eccentric coating (130), the cap coating (125) regulating elution of the therapeutic agent from the eccentric coating (130).

6. The coated stent of any of the preceding claims further comprising a cap coating (125) disposed on the eccentric coating (130).

7. The coated stent of claim 6 wherein the cap coating (125) is of substantially uniform thickness.

8. The coated stent of claim 6 or 7 wherein the cap coating (125) includes a therapeutic agent.

9. The coated stent of any of claims .6 to 8 wherein the cap coating (125) further comprises a first cap portion (126) and a second cap portion (127), the first cap portion (126) disposed on the first eccentric portion (131) and the second cap portion (127) disposed on the second eccentric portion (132).

10. The coated stent of claim 9 wherein the second cap portion (127) is thicker than the first cap portion (126).

11. The coated stent of claim 9 wherein the first cap portion (126) is thicker than the second cap portion (127).

12. The coated stent of any of claims 9 to 11 wherein the second cap portion (127) is biodegradable.

13. The coated stent of any of claims 9 to 12 wherein the first cap portion (126) includes one therapeutic agent and the second cap portion (127) includes a different therapeutic agent.

14. The coated stent according to any of the preceding claims, wherein the polymer/drug solution is cured to obtain the continuous eccentric layer (130).

15. The coated stent according to any of claims 1 to 13, wherein the polymer/drug solution is dried to obtain the continuous eccentric layer (130).

16. A method for producing a coated stent comprising:
providing a stent (120);
mixing a polymer and a therapeutic agent with a solvent to form a polymer/drug solution; and
applying the polymer/drug solution to the stent (120) as a continuous eccentric layer.;

17. The method of claim 16 wherein the stent has a stent outer diameter, and applying the polymer/drug solution further comprises:
mounting the stent on a coating fixture (198) having a fixture mandrel (200) inside the stent (120); and
spraying the polymer/drug solution at the stent outer diameter in the direction of the fixture mandrel (200).

18. The method of claim 16 wherein the stent has a stent outer diameter and a stent inner diameter, and applying the polymer/drug solution further comprises:
applying the polymer/drug solution to the stent inner diameter with an inner pad (274); and
applying the polymer/drug solution to the stent outer diameter with an outer pad (280).

19. The method of claim 16 wherein applying the polymer/drug solution to the stent as an eccentric layer further comprises applying the polymer/drug solution by an application method selected from the group consisting of painting, spraying, dipping, wiping, electrostatic deposition, vapor deposition, epitaxial growth, and combinations thereof.

20. The method of any of claims 16 to 19, further comprising curing the eccentric layer to form an eccentric coating.

21. The method of any of claims 16 to 19, further comprising drying the eccentric layer to form an eccentric coating.

22. The method of any of claims 16 to 21 further comprising:
mixing a polymer with a solvent to form a polymer solution;
applying the polymer solution to the eccentric coating as a cap layer; and
curing the cap layer to form a cap coating.

23. The method of claim 22 wherein the stent has a stent outer diameter and a stent inner diameter, and applying the polymer solution further comprises:
applying the polymer solution to the stent inner diameter with an inner pad (274); and
applying the polymer solution to the stent outer diameter with an outer pad (280).

24. The method of claim 22 wherein applying the polymer solution further comprises applying the polymer solution by an application method selected from the group consisting of painting, spraying, dipping, wiping, electrostatic deposition, vapor deposition, epitaxial growth, and combinations thereof.

## Patentansprüche

1. Beschichteter Stent, umfassend:
einen Stent (120), wobei der Stent einen Stentinnendurchmesser und einen Stentaußendurchmesser hat; und
eine exzentrische Beschichtung (130), die eine durchgehende exzentrische Schicht umfasst, gewonnen durch Aufbringen einer Polymer/Arzneimittel-Lösung auf den Stent, wobei die exzentrische Beschichtung (130) einen ersten exzentrischen Abschnitt (131) und einen zweiten exzentrischen Abschnitt (132) hat, wobei der erste exzentrische Abschnitt (131) auf dem Stentinnendurchmesser angeordnet ist und der zweite exzentrische Abschnitt (132) auf dem Stentaußendurchmesser angeordnet ist;
wobei der erste exzentrische Abschnitt (131) und der zweite exzentrische Abschnitt (132) unterschiedliche Dicken haben.

2. Beschichteter Stent nach Anspruch 1, wobei der zweite exzentrische Abschnitt (132) dicker als der erste exzentrische Abschnitt (131) ist.

3. Beschichteter Stent nach Anspruch 1, wobei der erste exzentrische Abschnitt (131) dicker als der zweite exzentrische Abschnitt (132) ist.

4. Beschichteter Stent nach einem der vorhergehenden Ansprüche, wobei die exzentrische Beschichtung (130) einen therapeutischen Wirkstoff beinhaltet.

5. Beschichteter Stent nach Anspruch 4, der ferner eine Deckbeschichtung (125) umfasst, die auf der exzentrischen Beschichtung (130) angeordnet ist, wobei die Deckbeschichtung (125) die Elution des therapeutischen Wirkstoffs aus der exzentrischen Beschichtung (130) reguliert.

6. Beschichteter Stent nach einem der vorhergehenden Ansprüche, der ferner eine Deckbeschichtung (125) umfasst, die auf der exzentrischen Beschichtung (130) angeordnet ist.

7. Beschichteter Stent nach Anspruch 6, wobei die Deckbeschichtung (125) von im Wesentlichen gleichmäßiger Dicke ist.

8. Beschichteter Stent nach Anspruch 6 oder 7, wobei die Deckbeschichtung (125) einen therapeutischen Wirkstoff beinhaltet.

9. Beschichteter Stent nach einem der Ansprüche 6 bis 8, wobei die Deckbeschichtung (125) ferner einen ersten Deckabschnitt (126) und einen zweiten Deckabschnitt (127) umfasst, wobei der erste Deckabschnitt (126) auf dem ersten exzentrischen Abschnitt (131) angeordnet ist und der zweite Deckabschnitt (127) auf dem zweiten exzentrischen Abschnitt (132) angeordnet ist.

10. Beschichteter Stent nach Anspruch 9, wobei der zweite Deckabschnitt (127) dicker als der erste Deckabschnitt (126) ist.

11. Beschichteter Stent nach Anspruch 9, wobei der erste Deckabschnitt (126) dicker als der zweite Deckabschnitt (127) ist.

12. Beschichteter Stent nach einem der Ansprüche 9 bis 11, wobei der zweite Deckabschnitt (127) biologisch abbaubar ist.

13. Beschichteter Stent nach einem der Ansprüche 9 bis 12, wobei der erste Deckabschnitt (126) einen therapeutischen Wirkstoff beinhaltet und der zweite Deckabschnitt (127) einen unterschiedlichen therapeutischen Wirkstoff beinhaltet.

14. Beschichteter Stent nach einem der vorhergehenden Ansprüche, wobei die Polymer/Arzneimittel-Lösung ausgehärtet wird, um die durchgehende exzentrische Schicht (130) zu erhalten.

15. Beschichteter Stent nach einem der Ansprüche 1 bis 13, wobei die Polymer/Arzneimittel-Lösung getrocknet wird, um die durchgehende exzentrische Lage (130) zu erhalten.

16. Verfahren zum Herstellen eines beschichteten Stents, umfassend:
Bereitstellen eines Stents (120);
Mischen eines Polymers und eines therapeutischen Wirkstoffs mit einem Lösungsmittel, um eine Polymer/Arzneimittel-Lösung zu bilden; und
Aufbringen der Polymer/Arzneimittel-Lösung auf den Stent (120) als eine durchgehende exzentrische Schicht.

17. Verfahren nach Anspruch 16, wobei der Stent einen Stentaußendurchmesser hat und das Aufbringen der Polymer/Arzneimittel-Lösung ferner umfasst:
Befestigen des Stents auf einer Beschichtungshalterung (198), die einen Halterungsdorn (200) innerhalb des Stents (120) hat; und
Sprühen der Polymer/Arzneimittel-Lösung an dem Stentaußendurchmesser in die Richtung des Halterungsdorns (200) .

18. Verfahren nach Anspruch 16, wobei der Stent einen Stentaußendurchmesser und einen Stentinnendurchmesser hat und das Aufbringen der Polymer/Arzneimittel-Lösung ferner umfasst:
Aufbringen der Polymer/Arzneimittel-Lösung auf den Stentinnendurchmesser mit einem inneren Kissen (274); und
Aufbringen der Polymer/Arzneimittel-Lösung auf den Stentaußendurchmesser mit einem äußeren Kissen (280).

19. Verfahren nach Anspruch 16, wobei das Aufbringen der Polymer/Arzneimittel-Lösung auf den Stent als eine exzentrische Schicht ferner umfasst, die Polymer/Arzneimittel-Lösung durch ein Aufbringungsverfahren aufzubringen, das ausgewählt ist aus einer Gruppe, die aus Pinseln, Sprühen, Eintauchen, Wischen, elektrostatischem Abscheiden, Gasphasenabscheidung, Epitaxie und Kombinationen davon besteht.

20. Verfahren nach einem der Ansprüche 16 bis 19, das ferner umfasst, die exzentrische Lage auszuhärten, um eine exzentrische Beschichtung zu bilden.

21. Verfahren nach einem der Ansprüche 16 bis 19, das ferner umfasst, die exzentrische Lage zu trocknen, um eine exzentrische Beschichtung zu bilden.

22. Verfahren nach einem der Ansprüche 16 bis 21, das ferner umfasst:
Mischen eines Polymers mit einem Lösungsmittel, um eine Polymer-Lösung zu bilden;
Aufbringen der Polymer-Lösung als eine Deckschicht auf die exzentrische Beschichtung; und
Aushärten der Deckschicht, um eine Deckbeschichtung zu bilden.

23. Verfahren nach Anspruch 22, wobei der Stent einen Stentaußendurchmesser und einen Stentinnendurchmesser hat und das Aufbringen der Polymer-Lösung ferner umfasst:
Aufbringen der Polymer-Lösung auf den Stentinnendurchmesser mit einem inneren Kissen (274); und
Aufbringen der Polymer-Lösung auf den Stentaußendurchmesser mit einem äußeren Kissen (280).

24. Verfahren nach Anspruch 22, wobei das Aufbringen der Polymer-Lösung ferner umfasst, die Polymer-Lösung durch ein Aufbringungsverfahren aufzubringen, das ausgewählt ist aus einer Gruppe, die aus Pinseln, Sprühen, Eintauchen, Wischen, elektrostatischem Abscheiden, Gasphasenabscheidung, Epitaxie und Kombinationen davon besteht.

## Revendications

1. Stent plaqué comportant :
un stent (120), ce stent ayant un diamètre intérieur de stent et un diamètre extérieur de stent ; et
un placage excentrique (130) comprenant une couche excentrique continue obtenue en appliquant une solution de polymère/médicament sur le stent, le placage excentrique (130) ayant un premier segment excentrique (131) et un second segment excentrique (132), le premier segment excentrique (131) étant disposé sur le diamètre intérieur du stent et le second segment excentrique (132) étant disposé sur le diamètre extérieur du stent ;
le premier segment excentrique (131) et le second segment excentrique (132) ayant des épaisseurs différentes.

2. Stent plaqué selon la revendication 1, dans lequel le second segment excentrique (132) est plus épais que le premier segment excentrique (131).

3. Stent plaqué selon la revendication 1, dans lequel le premier segment excentrique (131) est plus épais que le second segment excentrique (132).

4. Stent plaqué selon une des revendications précédentes, dans lequel le placage excentrique (130) contient un agent thérapeutique.

5. Stent plaqué selon la revendication 4, comprenant en outre un placage de calotte (125) disposé sur le placage excentrique (130), le placage de calotte (125) régulant l'élution de l'agent thérapeutique depuis le placage excentrique (130).

6. Stent plaqué selon l'une quelconque des revendications précédentes, comprenant en outre un placage de calotte (125) disposé sur le placage excentrique (130).

7. Stent plaqué selon la revendication 6, dans lequel le placage de calotte (125) a une épaisseur sensiblement uniforme.

8. Stent plaqué selon la revendication 6 ou 7, dans lequel le placage de calotte (125) contient un agent thérapeutique.

9. Stent plaqué selon l'une quelconque des revendications 6 à 8, dans lequel le placage de calotte (125) comprend en outre un premier segment de calotte (126) et un second segment de calotte (127), le premier segment de calotte (126) étant disposé sur le premier segment excentrique (131) et le second segment de calotte (127) étant disposé sur le second segment excentrique (132).

10. Stent plaqué selon la revendication 9, dans lequel le second segment de calotte (127) est plus épais que le premier segment de calotte (126).

11. Stent plaqué selon la revendication 9, dans lequel le premier segment de calotte (126) est plus épais que le second segment de calotte (127).

12. Stent plaqué selon l'une quelconque des revendications 9 à 11, dans lequel le second segment de calotte (127) est biodégradable.

13. Stent plaqué selon l'une quelconque des revendications 9 à 12, dans lequel le premier segment de calotte (126) contient un agent thérapeutique et le second segment de calotte (127) contient un agent thérapeutique différent.

14. Stent plaqué selon l'une quelconque des revendications précédentes, dans lequel la solution polymère/médicament est cuite pour obtenir la couche excentrique continue (130).

15. Stent plaqué selon l'une quelconque des revendications 1 à 13, dans lequel la solution polymère/médicament est séchée pour obtenir la couche excentrique continue (130).

16. Procédé de production d'un stent plaqué comprenant :
le fait de se procurer un stent (120) ;
le mélange d'un polymère et d'un agent thérapeutique avec un solvant pour former une solution polymère/médicament ; et
l'application de la solution polymère/médicament sur le stent (120) sous forme d'une couche excentrique continue (130).

17. Procédé selon la revendication 16, dans lequel le stent a un diamètre extérieur de stent et l'application de la solution polymère/médicament comprend en outre :
le montage du stent sur une fixation de placage (198) comportant un mandrin de fixation (200) à l'intérieur du stent (120) ; et
la pulvérisation de la solution polymère/médicament sur le diamètre extérieur en direction du mandrin de fixation (200).

18. Procédé selon la revendication 16, dans lequel le stent a un diamètre extérieur de stent et un diamètre intérieur de stent et l'application de la solution polymère/médicament comprend en outre :
l'application de la solution polymère/médicament sur le diamètre intérieur du stent avec un tampon intérieur (274) ; et
l'application de la solution polymère/médicament sur le diamètre extérieur du stent avec un tampon extérieur (280).

19. Procédé selon la revendication 16, dans lequel l'application de la solution polymère/médicament sur le stent sous forme de couche excentrique comprend en outre l'application de la solution polymère/médicament par un procédé d'application sélectionné parmi le groupe composé de la peinture, du trempage, de l'essuyage, du dépôt électrostatique, du dépôt en phase vapeur, de la croissance épitaxiale et leurs combinaisons.

20. Procédé selon l'une quelconque des revendications 16 à 19, comprenant en outre la cuisson de la couche excentrique pour former un placage excentrique.

21. Procédé selon l'une quelconque des revendications 16 à 19, comprenant en outre le séchage de la couche excentrique pour former un placage excentrique.

22. Procédé selon l'une quelconque des revendications 16 à 21, comprenant en outre :
le mélange d'un polymère avec un solvant pour former une solution polymérique ;
l'application de la solution polymérique sur le placage excentrique sous forme de couche de calotte ; et
la cuisson de la couche de calotte pour former un placage de calotte.

23. Procédé selon la revendication 22, dans lequel le stent a un diamètre intérieur de stent et un diamètre extérieur de stent et l'application de la solution polymérique comprend en outre :
l'application de la solution polymérique sur le diamètre intérieur du stent avec un tampon intérieur (274) ; et
l'application de la solution polymérique sur le diamètre extérieur du stent avec un tampon extérieur (280).

24. Procédé selon la revendication 22, dans lequel l'application de la solution polymérique comprend en outre l'application de la solution polymérique par un procédé d'application sélectionné parmi le groupe composé de la peinture, du trempage, de l'essuyage, du dépôt électrostatique, du dépôt en phase vapeur, de la croissance épitaxiale et leurs combinaisons.
